# DEMANDE DE BREVET EUROPEEN

(11) **EP 1 632 920 A1**
(43) Date de publication de la demande: **08.03.2006**
(21) Numéro de dépôt: 05300714.2
(22) Date de dépôt: 02.09.2005
(51) Int. Cl.: G08B 21/04, A61B 5/11

(54) **Dispositif portable de détection, d'alerte et de transmission d'informations relatives à une personne physique**

(30) Priorité: 02.09.2004 FR 0451961
(71) Demandeur: Burbaud, Thierry, 33290 Ludon-Medoc (FR)
(72) Inventeur: Burbaud, Thierry, 33290 Ludon-Medoc (FR)
(74) Mandataire: Fantin, Laurent

(57) **Abrégé**

L'objet de l'invention est un dispositif portable de détection, d'alerte et de transmission d'informations relatives à une personne physique, caractérisé en ce qu'il comprend au moins un circuit (12) comportant au moins un capteur (14) de choc, un capteur (16) de chute ainsi que des moyens (22) de transmission.

## Description

La présente invention concerne un dispositif portable de détection, d'alerte et de transmission d'informations relatives à une personne physique.

On connaît les contraintes imposées notamment par les personnes âgées tout autant que par les patients en hospitalisation à domicile qui ne sont actuellement pas résolus de façon satisfaisante.

Les personnes âgées lorsqu'elles sont à domicile se trouvent dans un environnement beaucoup plus satisfaisant que dans des centres organisés, au moins du point de vue psychologique. Ceci contribue grandement à prolonger leur bon état de santé ou du moins à retarder les symptômes de la vieillesse.

Par contre, l'âge étant là, les risques ne sont pas supprimés et il est nécessaire d'organiser une surveillance. Celle-ci ne peut pas être constante, ce qui mobiliserait une personne d'assistance pour une personne âgée voire plus car il faudrait pouvoir exercer la surveillance autant le jour que la nuit, ce qui est impossible.

Au mieux peut-il y avoir des visites régulières or les accidents tant médicaux que physiques et les chutes se produisent quasiment systématiquement entre deux passages.

Dans le cas de certaines affections, comment se prémunir contre la prise erronée de médicaments ou la non prise d'un traitement médical suivi, par exemple pour le rythme cardiaque.

Il existe bien sûr le téléphone ou des moyens portatifs de déclenchement en cas d'accident mais encore faut-il que la personne soit consciente d'une part et puisse avoir la capacité physique de s'en servir d'autre part.

Un autre milieu est celui des patients qui ont été hospitalisés. On sait que le milieu hospitalier doit être réservé en priorité aux patients qui le nécessitent.

D'une part les coûts d'hospitalisation sont élevés et d'autre part le milieu hospitalier n'est pas le lieu idéal de séjour pour des patients qui n'ont pas besoin de soins lourds et dont les soins peuvent être exercés à domicile.

Ces séjours en milieu hospitalier exposent de plus en plus les patients à des maladies nosocomiales, surtout en état de faiblesse.

Du point de vue psychologique, des études et des constatations ont montré que les durées de rétablissement sont beaucoup plus courtes pour un patient dès qu'il réintègre son domicile, lorsque cela est possible.

Par contre, une surveillance à domicile reste souvent nécessaire en attendant un rétablissement complet.

Au sein même d'un service hospitalier, on constate que le personnel est en nombre de plus en plus réduit. Il existe bien entendu des moyens de contrôle et de surveillance techniquement très perfectionnés mais ils ne remplissent pas certaines fonctions de surveillance. En effet, il faut se déplacer dans la chambre du patient pour constater qu'il y a une alarme qui s'est déclenchée. En cas de chute d'un patient qui s'est déplacé ou qui est descendu de son lit, il n'est prévu aucun moyen de surveillance sauf les rondes ou lors des soins et traitements.

De même, pour des patients qui ne sont pas sous monitoring, le personnel effectue des rondes de nuit, réveille à chaque ronde le patient pour prendre la température, mesurer la fréquence cardiaque et constater que le patient est bien dans son lit et n'a pas chuté.

Le personnel doit assurer des missions plus sophistiquées que de la simple surveillance, ce qui occupe une partie non négligeable de son temps.

Le but de la présente invention est de proposer un dispositif portable de détection, d'alerte et de transmission d'informations relatives à une personne physique qui permet de remplir certaines tâches contraignantes de surveillance, qui assure une alerte au moment opportun, avec un réglage de certains paramètres en fonction du porteur, qui permet des transmissions à distance et qui est d'un prix autorisant des investissements en milieu hospitalier mais aussi des investissements par des particuliers.

Le dispositif selon l'invention est maintenant décrit en détail, suivant un mode de réalisation particulier, non limitatif.

Des schémas sont annexés, correspondant aux différentes configurations :
- la figure 1 montre un schéma de fonctionnement pour un porteur à domicile, et
- la figure 2 montre un schéma de fonctionnement pour un porteur en milieu hospitalier.

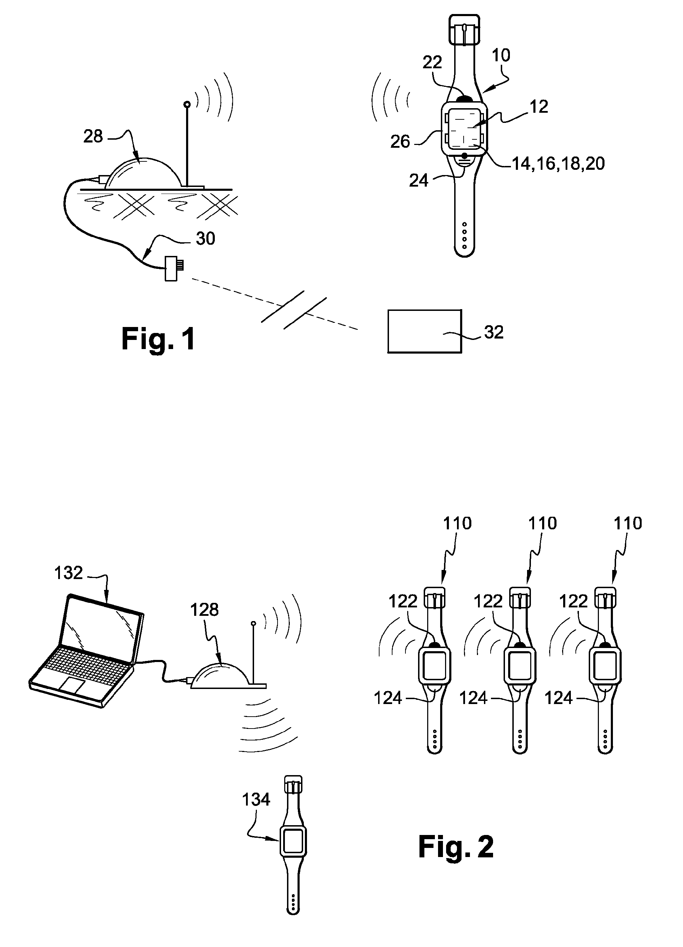

Le dispositif notamment montré sur la figure 1 de façon schématique et fonctionnelle comprend un boîtier 10 par exemple un boîtier du type montre bracelet.

Dans ce boîtier, on a intégré un circuit 12 comportant au moins un capteur 14 de choc, un capteur 16 de chute, un capteur 18 de température et un capteur 20 de tension et de fréquence cardiaque.

Il est aussi adjoint des moyens 22 de transmission.

De façon perfectionnée, on intègre dans ce boîtier des moyens 24 de communication vocale.

Le circuit est alimenté en énergie par une source 26 autonome, du type piles ou accus rechargeables avec une alerte en cas de puissance faible.

Le capteur 14 de choc et le capteur 16 de chute intègrent les valeurs données par des accéléromètres et des gyroscopes intégrés dans une centrale inertielle miniature.

Dès que les valeurs enregistrées correspondent à des accélérations supérieures à un seuil donné, on en déduit qu'il y a une chute et non un mouvement volontaire.

Par les gyroscopes de la centrale inertielle, on déduit la position dans l'espace du dispositif qui est lui-même solidaire des mouvements du porteur. On peut associer à ces valeurs de choc, un défaut de verticalité ou une variation importante de l'angle, au-delà d'un seuil, ce qui correspond à une chute.

Dès que des seuils préfixés sont dépassés, les alarmes déclenchent un signal. Ce signal est envoyé à travers les moyens 22 de transmission vers une base 28.

Ces moyens de transmission utilisent des ondes adaptées, et le système connu sous la dénomination commerciale "blue tooth" est parfaitement fonctionnel dans cette application, tant dans sa portée que dans ses coûts et dans ses qualités de transmission.

Dans le cas d'un particulier, c'est-à-dire une hospitalisation à domicile ou une surveillance d'une personne âgée, les moyens de transmission recourent ensuite à partir de la base 28 au réseau téléphonique 30 existant, soit filaire, soit cellulaire pour conduire les informations à un poste central 32.

A partir de ce poste central, le personnel de surveillance peut obtenir simultanément à l'alerte le type de facteur qui a déclenché cette alerte : chute, augmentation du rythme cardiaque ou augmentation de la tension, élévation de la température. De fait la gravité des faits ou la nature induit l'envoi d'un personnel adapté avec une urgence proportionnée.

Les moyens 24 de communication vocale comprennent un microphone et un haut parleur avec un contacteur de mise en service volontaire. Cet ensemble peut aussi être aussi mis en service simultanément à toute alerte ou à distance par le poste central.

De tels moyens permettent de savoir si le porteur est conscient ou inconscient. En effet, il est possible par le microphone d'écouter l'environnement sonore du porteur. En cas de porteur conscient, il est permis d'échanger et surtout le porteur sait qu'il a été entendu, ce qui est particulièrement rassurant.

Les différents seuils d'alerte peuvent aussi être programmés sur le dispositif car le rythme cardiaque ou la tension n'est pas le même pour tous et surtout les plages d'alerte ne sont pas les mêmes pour les différents porteurs.

Le personnel médical est à même d'indiquer au personnel de surveillance les seuils à considérer comme étant des alertes.

La programmation peut s'effectuer à l'aide de touches de façon connue, sans pour cela nécessiter une programmation lourde et fastidieuse.

Un verrouillage et/ou un code peuvent être prévus en sorte d'éviter les manoeuvres par des tiers non autorisés.

De même pour la fixation sur le poignet du porteur, il est aussi possible de prévoir une mise en place avec un verrouillage en sorte d'éviter le retrait intempestif volontaire ou involontaire du porteur et l'oubli de remise en place, ce qui annihilerait toutes les potentialités du dispositif.

De fait, le boîtier doit nécessairement être étanche, ce qui est réalisable aisément, surtout qu'il s'agit généralement d'une étanchéité à faible pression.

La lecture doit aussi être facilitée par un écran de dimensions adaptées ou avec des pictogrammes significatifs et instinctifs permettant une manoeuvre quelle que soit la situation.

Dans la situation d'un service hospitalier commun, l'agencement est strictement identique, avec une base, les mêmes éléments portant les mêmes numéros augmentés de 100.

Une des différences est que le nombre de dispositifs est multiplié par autant de patients à surveiller. Quant à la base 128 elle est plutôt reliée à un ordinateur 132 qui sert de centralisation des informations. Cet ordinateur muni d'un écran affiche simultanément toutes les données d'au moins un dispositif en sorte de pouvoir les consulter à distance ou consulter celui qui aura déclenché une alarme.

Quand plusieurs dispositifs déclenchent une alarme, la personne soignante peut rapidement analyser la situation et classer les interventions par ordres de priorité.

Comme précédemment, la communication vocale est possible avec les mêmes avantages notamment celui de rassurer le patient à travers les moyens 124 de communication vocale.

Selon un perfectionnent de l'invention, la personne soignante est également munie de moyens 134 portables d'information et d'intervention, qui reçoivent les mêmes informations que l'ordinateur en sorte de pouvoir capter les informations en provenance des dispositifs portés par les patients, ceci lorsque la personne soignante est déjà en intervention et/ou en ronde de soins et/ou absente du lieu de surveillance.

Ces moyens portables permettent de rester en contrôle continu sans pour cela immobiliser une personne.

De plus, on constate un gain considérable de temps et d'énergie pour le personnel soignant car les paramètres transmis renseignent sur le type d'alarme et sur la cause probable de l'appel, en tout cas sur le degré d'urgence.

Lorsqu'un dispositif d'appel est prévu au chevet d'un malade, la nature de la demande n'est absolument pas formalisée. La personne soignante doit aller auprès du patient pour constater qu'il s'agit, la plupart du temps d'une requête d'une très faible importance. Il s'avère même que dans certains cas, il faut revenir au point de départ pour prendre un soin, un objet, ce qui augmente considérablement les trajets et le temps perdu.

## Revendications

1. Dispositif portable de détection, d'alerte et de transmission d'informations relatives à une personne physique, **caractérisé en ce qu'**il comprend au moins un circuit (12) comportant au moins un capteur (14) de choc, un capteur (16) de chute ainsi que des moyens (22) de transmission, des moyens (24) de communication vocale, l'ensemble étant disposé dans un boîtier (10) étanche muni d'une source (26) autonome avec une alerte en cas de puissance faible.

2. Dispositif portable de détection, d'alerte et de transmission d'informations selon la revendication 1, **caractérisé en ce qu'**il comprend en outre un capteur (18) de température et un capteur (20) de fréquence et de tension cardiaques.

3. Dispositif portable de détection, d'alerte et de transmission d'informations selon la revendication 1 ou 2, **caractérisé en ce que** les capteurs (14) de choc et (16) de chute intègrent une centrale inertielle.

4. Dispositif portable de détection, d'alerte et de transmission d'informations selon l'une quelconque des revendications précédentes, **caractérisé en ce que** les moyens (22, 122) de transmission incluent une base (28, 128) liée par le réseau téléphonique (30) à un poste central (32) ou à un ordinateur (132) pour conduire les informations.

5. Dispositif portable de détection, d'alerte et de transmission d'informations selon l'une quelconque des revendications précédentes, **caractérisé en ce que** les moyens de transmission comprennent des moyens (134) portables d'information et d'intervention.
